# EUROPEAN PATENT SPECIFICATION

(11) **EP 4 530 528 B1**
(45) Date of publication and mention of the grant of the patent: **28.10.2026**
(21) Application number: 24218481.0
(22) Date of filing: 03.04.2013
(51) Int. Cl.: A61B 18/04, F22B 1/28

(54) **INDUCTION COIL VAPOR GENERATOR**
INDUKTIONSSPULENDAMPFERZEUGER
GÉNÉRATEUR DE VAPEUR À BOBINE D'INDUCTION

(30) Priority: 03.04.2012 US 201261619818 P
(43) Date of publication of application: 02.04.2025
(62) Document number(s) of the earlier application(s) in accordance with Art. 76 EPC: 20199957.0 / 3 777 738; 13771921.7 / 2 833 815
(73) Proprietor: Boston Scientific Scimed, Inc., Maple Grove, MN 55311 (US)
(72) Inventor: HOEY, Michael, Maple Grove, MN, 55369 (US); PAULOS, Stephanos, Maple Grove, MN, 55369 (US); HASTINGS, Roger Noel, Maple Grove, MN, 55369 (US)
(74) Representative: Pfenning, Meinig & Partner mbB

(56) References cited:
- CN-A- 101 803 947
- US-A1- 2002 078 956
- US-A1- 2008 217 325
- US-A1- 2010 204 688
- US-A1- 2011 264 176

## Description

### FIELD

This disclosure generally relates to vapor generation in medical instruments. More specifically, this disclosure relates to induction coil vapor generators in medical instruments.

### BACKGROUND

Benign prostatic hyperplasia (BPH) is a common disorder in middle-aged and older men, with prevalence increasing with age. At age 70, more than one-half of men have symptomatic BPH, and nearly 90% of men have microscopic evidence of an enlarged prostate. The severity of symptoms also increase with age with 27% of patients in the 60-70 age bracket having moderate-to-severe symptoms, and 37% of patients in their 70's suffering from moderate-to-severe symptoms.

The prostate early in life is the size and shape of a walnut and weighs about 20 grams. Prostate enlargement appears to be a normal process. With age, the prostate gradually increases in size to twice or more its normal size. The fibromuscular tissue of the outer prostatic capsule restricts expansion after the gland reaches a certain size. Because of such restriction on expansion, the intracapsular tissue will compress against and constrict the prostatic urethra thus causing resistance to urine flow.

BPH is typically diagnosed when the patient seeks medical treatment complaining of bothersome urinary difficulties. The predominant symptoms of BPH are an increase in frequency and urgency of urination. BPH can also cause urinary retention in the bladder which in turn can lead to lower urinary tract infection (LUTI). In many cases, the LUTI then can ascend into the kidneys and cause chronic pyelonephritis, and can eventually lead to renal insufficiency. BPH also may lead to sexual dysfunction related to sleep disturbance or psychological anxiety caused by severe urinary difficulties. Thus, BPH can significantly alter the quality of life with aging of the male population.

BPH is the result of an imbalance between the continuous production and natural death (apoptosis) of the glandular cells of the prostate. The overproduction of such cells leads to increased prostate size, most significantly in the transitional zone which traverses the prostatic urethra.

In early stage cases of BPH, treatments can alleviate the symptoms. For example, alpha-blockers treat BPH by relaxing smooth muscle tissue found in the prostate and the bladder neck, which may allow urine to flow out of the bladder more easily. Such drugs can prove effective until the glandular elements cause overwhelming cell growth in the prostate.

More advanced stages of BPH, however, can only be treated by surgical or minimally invasive tissue ablation interventions. A number of methods have been developed using electrosurgical or mechanical extraction of tissue, and thermal ablation of intracapsular prostatic tissue. In many cases, such interventions provide only transient relief, and there often is significant peri-operative discomfort and morbidity.

Document US 2010/204688 A1 discloses an instrument and method for tissue thermotherapy including an inductive heating means to generate a vapor phase media that is used for interstitial, intraluminal, intracavity or topical tissue treatment. In one method, the vapor phase media is propagated from a probe outlet to provide a controlled vapor-to-liquid phase change in an interface with tissue to thereby apply ablative thermal energy delivery.

### SUMMARY OF THE DISCLOSURE

The invention is defined by the independent claim. Certain embodiments are specified in the dependent claims. Any examples, aspects, methods, embodiment and the like described herein and not falling within the scope of the claim are provided for illustrative purposes. An inductive vapor generator is provided, comprising an outer support assembly, a coil disposed around the outer support assembly, an inner assembly disposed within the outer support assembly, a plurality of microtubes disposed within the inner assembly, the microtubes being configured to receive a fluid from a fluid source, and a RF generator electrically coupled to the coil, the RF generator being configured to apply a current to the coil to inductively heat fluid in the plurality of microtubes to convert the fluid into a heated condensable vapor.

In some embodiments, the outer support assembly comprises a thermally insulating and electrically insulating tube.

In other embodiments, the inner assembly comprises a thermally conductive and electrically insulating tube.

In some embodiments, the generator comprises an air gap between the outer support assembly and the inner assembly.

In one embodiment, the plurality of microtubes comprises stainless steel microtubes.

In some embodiments, the inner assembly comprises a solid metal rod and the plurality of microtubes comprises an array of channels drilled through the solid metal rod.

In one embodiment, the outer support assembly has a length of less than 70mm.

In another embodiment, the outer support assembly has a diameter of less than 10mm.

A medical vapor delivery device is also provided, comprising a handle, a working shaft coupled to the handle, an induction vapor generator disposed in the handle and fluidly coupled to a fluid source and the working shaft, the induction vapor generator comprising, an outer support assembly, a coil disposed around the outer support assembly, an inner assembly disposed within the outer support assembly, a plurality of microtubes disposed within the inner assembly, a RF generator electrically coupled to the coil, the RF generator configured to apply a current to the coil, and a controller configured to apply a current to the coil to convert a fluid in the plurality of microtubes into a heated vapor inside the induction vapor generator for delivery of the heated vapor through the working shaft to human tissue.

In some embodiments, the device further comprises a treatment needle disposed near a distal portion of the working shaft.

In some embodiments, the outer support assembly comprises a thermally insulating and electrically insulating tube.

In other embodiments, the inner assembly comprises a thermally conductive and electrically insulating tube.

In some embodiments, the generator comprises an air gap between the outer support assembly and the inner assembly.

In one embodiment, the plurality of microtubes comprises stainless steel microtubes.

In some embodiments, the inner assembly comprises a solid metal rod and the plurality of microtubes comprises an array of channels drilled through the solid metal rod.

In one embodiment, the outer support assembly has a length of less than 70mm.

In another embodiment, the outer support assembly has a diameter of less than 10mm.

A method of generating a heated condensable vapor is also provided, comprising injecting a fluid into a plurality of microtubes disposed within an inner tube assembly, applying a current to a coil that is supported by an outer tube assembly, the inner tube assembly being disposed within the outer tube assembly, and inductively heating the fluid within the plurality of microtubes to convert the fluid to a heated condensable vapor.

In some embodiments, the method further comprises insulating the coil from heat with a gap disposed between the inner tube assembly and the outer tube assembly. In one embodiment, the gap comprises an air gap.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
Fig. 1 illustrates an induction coil vapor generator.
Fig. 2 shows one embodiment of a coiled magnetic wire of the vapor generator.
Fig. 3 shows one embodiment of quartz tubes used in the vapor generator.
Fig. 4 illustrates one embodiment of a pressure-fit cap of the vapor generator.
Fig. 5 illustrates one embodiment of a handheld vapor delivery device fitted with the induction coil vapor generator.
Fig. 6 is a flowchart illustrating one embodiment of an induction coil vapor generator system.
Figs. 7A-7C illustrate various matching networks for matching an RF generator to an induction vapor generator.
Figs. 8A-8C illustrate another embodiment of a vapor generator.

### DETAILED DESCRIPTION

The present disclosure describes methods and apparatus for treating BPH, comprising introducing a heated vapor interstitially into the interior of a prostate, wherein the vapor controllably ablates prostate tissue. This method can utilize vapor for applied energy of between 100 calories and 1,000 calories per lobe in an office-based procedure. In some embodiments, the system includes a vapor delivery mechanism that generates and delivers high quality heated water vapor. The system can utilize a vapor source configured to provide vapor having a temperature of at least 60° C, 80° C, 100° C, 120° C, or 140° C. In some embodiments, the vapor source can comprise an induction coil vapor generator disposed within a vapor delivery device, or alternatively, located outside of the vapor delivery device.

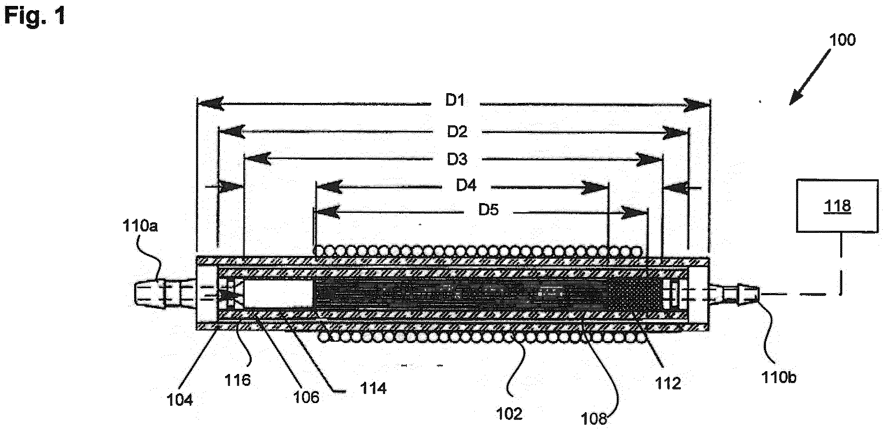

Fig. 1 illustrates a cutaway view of an induction coil vapor generator 100 configured to generate a high quality heated condensable vapor. The induction coil vapor generator can include an outer assembly of Litz wire or coiled magnet wire 102 supported by an outer assembly 104, and an inner assembly 106 disposed within the outer assembly. The generator can further include a plurality of metallic microtubes 108 disposed within the inner assembly. The microtubes can be coupled to a fluid source 118.

In some embodiments, the outer assembly can comprise an electrically insulating and thermally insulating material, such as fiberglass, or low density silicone, and the inner assembly can comprise an electrically insulating, thermally conductive material, such as aluminum nitride, alloys of iron including stainless steels, alloys of nickel, alloys of cobalt, quartz, glass, or a ceramic such as aluminum oxide. The outer assembly can be thermally insulating to prevent heat from damaging the coiled magnet wire or from damaging human tissue or other medical device components positioned exterior to the outer assembly. The inner assembly and the microtubes can be thermally conductive so as to inductively heat fluid to convert the fluid into a condensable vapor.

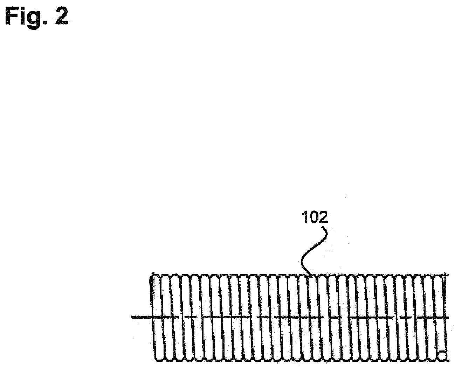

The coiled magnet wire can comprise any electrically insulated wire, such as insulated copper, silver, gold or aluminum wire used in electromagnets (magnet wire or Litz wire), and can be coupled to a RF generator that produces a current in the coiled magnet wire to generate an inductive field. This inductive field can then be used to heat the inner assembly and/or microtubes to convert fluid in the microtubes into a heated condensable vapor. In one embodiment, the coiled magnet wire comprises 16 gauge copper coil, or magnet wire in the diameter range of 10 AWG to 20 AWG. In another embodiment, the outer assembly is wrapped in magnet wire having 16 AWG and approximately 30.5 turns. The length of the tubes and the gauge and number of wraps of the wire can be adjusted, however, depending on the application and the desired size of the generator, for example with coils in the range of 10 to 100 turns wound with wire in the range 10 to 20 AWG. Fig. 2 shows another view of the coiled magnet wire 102 of Fig. 1.

Referring back to Fig. 1, the inner assembly can house a plurality of metallic microtubes 108, preferably having magnetic permeability larger than 1.5 at the operating frequency of the generator. The plurality of microtubes can comprise an array of 10 to 250 or more metallic microtubes, aligned in a parallel array configured to inductively heat and convert a precisely controlled quantity of sterile water or fluid into a superheated water vapor. Microtubes 108 may have outside diameters ranging from 0.5 mm to 2.5 mm, and inside diameters in the range of 0.25 to 2 mm. The microtubes 108 may be bundled together, preferably tightly enough so that they are in physical contact with each other. They may be physically joined with a metallic material such as solder, or the tubes may simply be long holes drilled through the length of a solid metallic rod.

The microtubes 108 can be encased in inner assembly 106, with O-ring supported pressure-fit caps 110a and 110b sealing both ends of the inner assembly. Cap 110a can be disposed on a distal, vapor emitting end of the generator 100, and cap 110b can be disposed on a proximal, fluid source connecting end of the generator. Liquid water or fluid can flow into the inner assembly and microtubes from a remote fluid source, such as fluid source 118, and the fluid can be inductively heated and converted into condensable vapor when a current is applied to the coiled magnet wire 102 (such as by a RF generator). The parallel array of the microtubes 108 in the inner assembly allows laminar flow of the water/vapor, minimizing back pressure on the fluid source. Heat is transferred efficiently from the inside and outside surfaces of the heated microtubes to the fluid flowing through and between the microtubes because of the large ratio of heated surface area to fluid volume in the microtubes.

In one embodiment, the microtubes can be aligned in parallel with the length of the coiled magnet wire, thereby making them parallel to the inductive magnetic field that is generated when current is applied to the coil. When the tubes are aligned in this fashion, current flows around the circumference of the tubes maximizing the ability of each tube to be heated when an inductive magnetic field is created. As fluid enters the inner assembly and is converted to vapor, the fluid and vapor passes through and between the tubes, thereby contacting both their inner and outer surfaces during the super heating travel from one end of the assembly to the other.

In one embodiment, the idealized expansion ratio of a volume of liquid water converted to pure water vapor is 1760x. This liquid-to-vapor expansion introduces significant mechanical forces even at the small volumes utilized in the inductive vapor generator described above. The most notable mechanical force is back pressure on the fluid source that delivers fluid to the inner assembly and microtubes. Since vapor expansion in a container has no preferred direction, the vapor produced can push backwards as well as forward. The arrangement of the microtubes can minimize the adverse back pressure to allow a syringe pump of normal strength to be used. The microtubes promote laminar flow and also prevent a "core" of liquid water from slipping through the generator by increasing the amount of surface area the water is in contact with. Enhanced contact of the water with the tube surfaces may be achieved by roughening or patterning the tube surfaces to encourage turbulent flow of the water.

Referring still to Fig. 1, in some embodiments, a gap 116 can be provided between the outer assembly and the inner assembly. In this embodiment, the outer assembly 104 is placed over the inner assembly 106, which provides support for the inductive coil, and the gap provides insulation between the inner assembly and both the outer assembly and inductive coil. The air gap can prevent damage to the inductive coil from being secondarily heated by the inner assembly, and retains heat in the inner assembly for more efficient transfer of heat to the water. The gap 116 can comprise only air, or alternatively, the gap can be filled with a gas, fluid, or gel with a low thermal conductivity, such as argon. In this embodiment, since the gap 116 can be used to thermally protect the outer assembly and the coiled magnet wire, it may not be necessary for the outer assembly to be thermally insulating. Thus, in this embodiment, the outer assembly can also comprise quartz, glass, ceramic, in addition to the materials listed above.

In an alternative embodiment, the coiled magnet wire 102 may be wound directly onto the inner assembly 106, placing the outer coil in direct thermal contact with the inner coil assembly. As long as water is being converted to steam in the inner coil assembly 106, the inner coil, and in this embodiment the outer coil, will be maintained at the boiling temperature of water during therapy delivery. Providing that the insulation of the coiled magnet wire or Litz wire of the outer coil can operate at the boiling temperature of water, this is an acceptable embodiment. Commonly used insulation materials can operate to at least 200°C, well above the boiling temperature of water, even at elevated pressures experienced within the heating element. This embodiment may insure repeatable temperatures during therapy and between therapy applications.

The inner assembly further comprises a sintered filter 112, configured to mechanically aerosolize the fluid as it enters the inner assembly and also to impede backflow, and a spacer tube 114, configured to further increase the ratio of vapor to liquid water before the heated condensable vapor leaves the generator. The sintered filter can comprise a porous disc made of, for example, 316 stainless steel and including very small openings on the order of 10 microns. The spacer tube 114 can comprise a stainless steel tube and can be relatively short compared to the length of the tubes. For example, in one embodiment, the spacer tube is up to approximately 8.5mm long or 10-15% of the length of the insulating tubes.

In some embodiments, the outer assembly 104 can have a length D1 ranging from 40mm to 100mm and a diameter ranging from 4mm to 25mm. The inner assembly 106 can have a length D2 ranging from 35mm to 95mm and a diameter ranging from 2mm to 23mm. The coiled magnet wire 102 can be wound along a length D5 of the outer assembly, ranging from the full length of the outer assembly to only the length of the microtubes 108 (microtube length illustrated by D4). D3 in Fig. 1 illustrates the total length that fluid/vapor travels between end caps 110b and 110a.

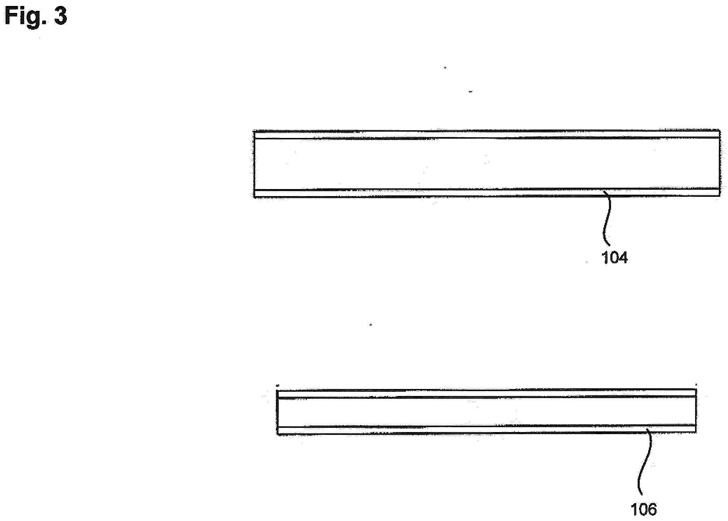

Fig. 3 illustrates outer and inner assemblies 104 and 106 separate from the rest of the generator assembly. In embodiments incorporating an air gap, when the inner assembly is positioned within the outer assembly, the two tubes can be separated by an air gap of up to 1-5mm.

In one embodiment, the inner and outer assemblies can be fused quartz, having a low coefficient of thermal expansion, on the order of 0.55x10⁻⁸/°C, and a temperature strain point of approximately 1070°C. The quartz tubes have a high resistance to thermal shock, and relatively low thermal conductivity (approximately 0.0033 cal cm⁻¹sec⁻¹°C and low dielectric loss factor (<0.0004 at 20°C, 1MHz). The quartz tubes also have a high tensile strength (approximately 7,000 psi) and a very high compressive strength (approximately 11.1x10⁶ psi).

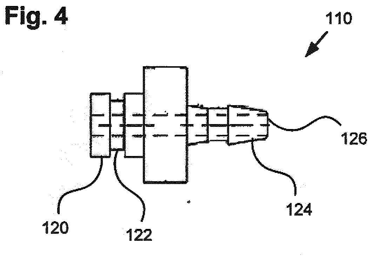

Fig. 4 shows a side view of a pressure fit cap 110 (e.g., such pressure fit caps 110a and 110b from Fig. 1). The pressure fit cap can include a proximal portion 120 that can include an o-ring seal 122 and a distal portion 124 that can either connect to the fluid source or pass the exiting vapor. Pressure fit 110 can also include lumens 116 running through the caps to allow for hookup to a fluid source on a proximal end of the generator, and to allow for vapor to exit on a distal end of the generator from the internal assembly. The proximal portion 120 and o-ring seal 122 are configured to mate with an internal surface of the internal assembly (e.g., internal assembly 106 from Fig. 1). The o-ring seal is configured to seal the internal assembly off from the rest of the generator.

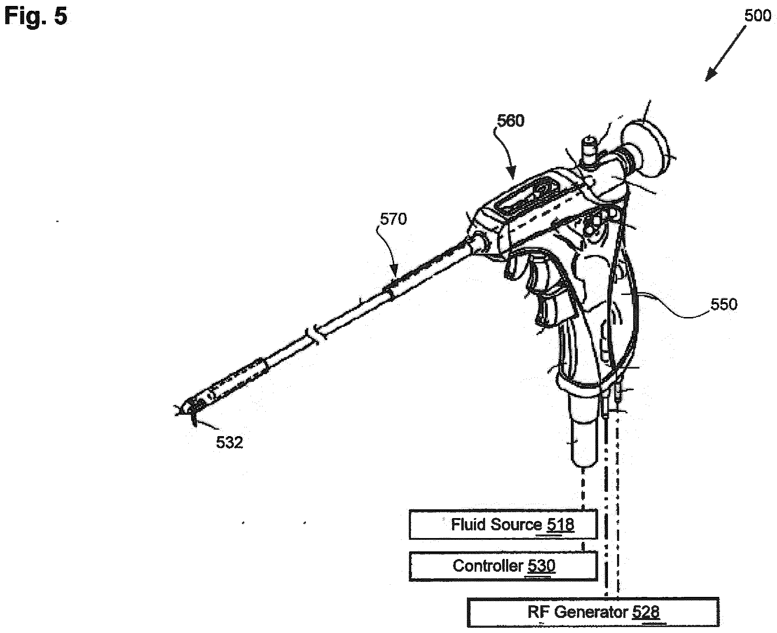

The inductive generators described herein can be designed to fit into a handheld vapor delivery device, and integrate with a controller 530, a fluid source 518, and a radiofrequency (RF) generator 528. Fig. 5 illustrates a handheld vapor delivery device 500 fitted with the induction coil vapor generator 100 of Fig. 1. More details of a handheld vapor delivery device as shown in Fig. 5 can be found in US Application No. 13/072,573, which is incorporated herein by reference. In some embodiments, as shown, the vapor generator can be disposed within the device. The vapor generator can be disposed in the handle, as shown by reference 550, or the vapor generator can be disposed in a top portion of the device, as shown by reference 560. In another embodiment, the vapor generator can be disposed inside a shaft of the delivery device, as shown by reference 570. The heated vapor formed by the inductive vapor generator in the device 500 can be coupled to a working shaft or treatment needle 532 of the device to deliver the vapor to human tissue.

In some embodiments, the device 500 is configured for transurethral access to the prostate of the patient. When the distal end of the device is positioned in the prostatic urethra near the prostate, needle 532 can be advanced into the prostate, and vapor generated in the inductive vapor generator can be delivered to the prostate to treat the prostate tissue.

In other embodiments, the vapor generator can be disposed external to the device 500 and can be configured to deliver vapor to the device. A proximal portion of the vapor generator can be fluidly coupled to a fluid source. A distal portion of the vapor generator can be fluidly coupled to the delivery device.

Inductive heating provides fast, consistent heat and can be used in a variety of applications. Inductive heating occurs when an electrically conducting object is exposed to a rapidly changing magnetic field. This is caused by an outer component such as a coil, through which a large high frequency electric current flows. This outer component experiences minimal heating itself due to its low resistance. The passage of current through this coil generates a very intense and rapidly changing magnetic field in the space within the coil. The magnetic field generated by the coil is dependent on the coil current multiplied by the number of coil turns per unit length (ampere turns per meter).

The alternating magnetic field induces a current flow in the inner component, or the metal being heated. The arrangement of the coil and the inner component can be thought of as an electrical transformer, but one in which electrical power transfer is intended to be inefficient, changing the "wasted" electrical energy to heat energy. The coil is the primary winding receiving RF energy from the RF generator and the inner component is like a single turn secondary winding that is short-circuited. This causes high currents (called eddy currents) to flow through the inner component causing it to heat up.

In addition to this, the high frequency used for induction heating gives rise to a phenomenon called skin effect. This skin effect forces the induced alternating current to flow in a thin layer towards the surface of the inner component. The skin effect increases the effective resistance of the metal to the passage of the large current. Therefore it greatly increases the heating effect caused by the current induced in the workpiece. On the other hand, the skin effect may adversely increase the resistance of the coiled magnet wire 102.

For ferrous metals like iron and some steels, the magnetic permeability of the inner coil material can enhance or magnify the magnetic field generated by the coiled magnet wire 102, thereby increasing the magnitude of the eddy currents and inductive heating of the inner coil for a given current flowing in the outer coiled wire. There is an additional heating mechanism that takes place in ferromagnetic materials at the same time as the eddy currents mentioned above. These intense magnetic fields inside the coil repeatedly magnetize and de-magnetize the iron crystals. This rapid flipping of the magnetic domains causes considerable friction and heating inside the material. Heating due to this mechanism is known as Hysteresis loss. For these reasons, ferrous materials having magnetic properties lend themselves more easily to induction heating than those without magnetic properties.

Unlike direct resistive heating, the current coming directly from the power source never directly contacts the material being heated. Instead, it is transformed first to a magnetic field, then as that magnetic field affects the conduction in the inner component, back to a self-contained secondary electric circuit.

Direct resistive heating is also capable of making water vapor, in some applications. However, arcing and plating are inescapable by-products of using direct resistive heating and the consequences (deterioration of electrodes, etc.) create great difficulties in controlling such vapor for precision applications.

Several parameters must be balanced in an induction generator design to improve the quality of the vapor being generated. These parameters include, but are not limited to: magnet wire coil impedance, frequency of driving RF energy, composition of materials being heated, geometry of internal components, management of mechanical forces, and RF source impedance matching (including C-matching network).

Inductance in the coil can be increased by adding more turns to the wire coil, increasing the coil diameter, and shortening the length of the coil. However, increasing inductions via each of these examples may also come with some consequences. For example, adding more turns to the wire coil (where the coil maintains the same coil length) requires a thinner coil wire, which increases the resistive heating in the outer coil component. This is one of the consequences that the use of inductive heating is designed to prevent.

In one example of the coil in Fig. 2, constructed with 16 AWG magnet wire, the resistance is about 0.013 Ohms at DC. The resistance of copper increases with frequency due to the skin effect. However, if the 16 AWG wire is made from insulated strands of smaller diameter wire, i.e. Litz wire, such that the diameter of the individual strands of wire are smaller than twice the skin depth at the operating frequency, this increase of resistance with frequency can be avoided. When a typical rms current of about 17 amps is applied, the Ohmic heat generated in the coil is about 4 Watts, which can be readily dissipated in the hand piece of the device of Fig. 5. Other coil designs may create excessive heating of the coil. The resistance of the coil increases with increasing temperature, further increasing heat generation for a fixed current and fixed magnetic induction within the coil. If a constant induction is maintained, coil temperatures may rapidly rise beyond the temperature for which the coil insulation degrades. If a fixed input power is maintained, resistive heating in the outer coil reduces the amount of electrical energy being used to generate the required magnetic field, causing in turn less ability to heat the inner component. Coil resistance can be minimized to acceptable levels if coil resistance can be at least 100 times smaller than inductive resistance. When the coil of Fig. 2 is operated at 250 kHz, the ratio of inductive reactance to coil resistance can be larger than unity and may be as large as about 280.

A larger coil diameter causes the generator unit to increase in size, and reduces the magnetic coupling to the inner heated component which can cause efficiency reduction. If the inner heated component is made larger to compensate, the vapor inside this larger space must be reduced proportionally more to enter the treatment needle, which can cause turbulent flow. This also causes back pressure on the syringe pump delivering liquid water to be converted to vapor, and may even cause condensation.

Shortening the length of the assembly means a shorter length for the inner, heated component. As the ability to convert liquid water to vapor is directly proportional to the surface area of the heated inner component that the water can contact, and a shorter assembly of the same design will always have less surface area, a shorter assembly will be less able to completely and consistently create vapor.

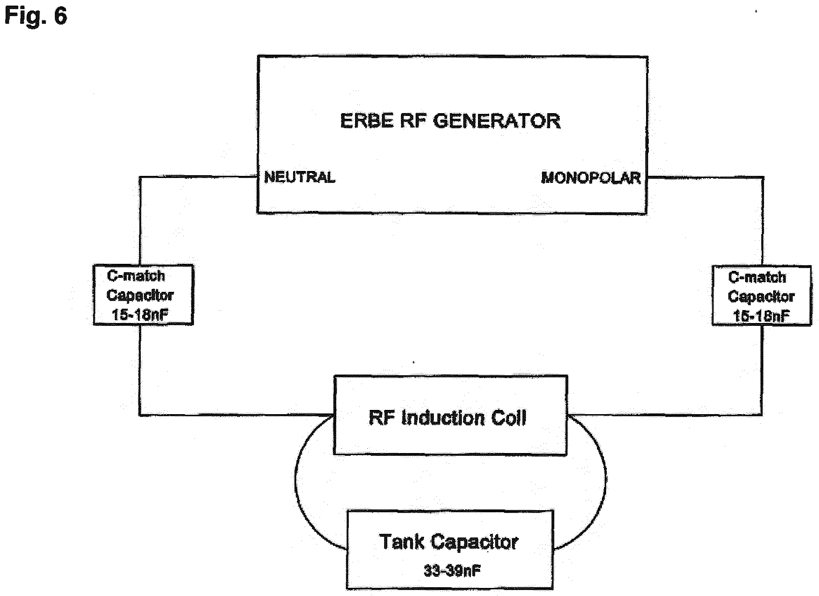

Fig. 6 illustrates a schematic diagram of a system including an RF generator, a C-matching network, and an induction coil (as described above). Greater inductive power results in more inductive heating if all other factors are constant, and greater inductive power can be achieved by applying more current to the generator. However, if the inductive coil portion of the load acts to absorb power, it can cause resistive heating in the coil. If the coil has low impedance, however, the residual power exiting the coil will impact power coming in the opposite direction during each cycle of the polarity (vector) shift. The trade-off between these factors leads to the importance of a proper impedance matching network.

In electronics, impedance matching is the practice of designing the input impedance of an electrical load or the output impedance of its corresponding signal source in order to maximize the power transfer and/or minimize reflections from the load. In the vapor generator described above, a higher frequency is advantageous since only the inductive microtubes need to be heated in order to convert liquid to vapor. A high frequency combined with a load resistance in the wire coil causes the impedance of the generator to become primarily reactive, which calls for complex conjugate mapping and C-matching.

In the generator above, the wire coil is made to resonate at the intended operating frequency by way of a capacitor placed in parallel with the coil. This parallel resonance recaptures the current exiting the coil, both preventing the current from being reflected against incoming current in the opposite direction during polarity shift and magnifying the current through the work coil compared to the output current capability of the RF generator alone. In this case, the RF generator only has to provide the part of the load current that actually does real work and is lost by converting electrical energy to heat, not the full circulating current in the coil.

The parallel resonant circuit can make a tenfold reduction in the current that must be supported by the RF generator and the wires connecting it to the coil. Conduction losses are typically proportional to current squared, so a tenfold reduction in load current represents a significant saving in conduction losses in the RF generator and associated wiring.

In a parallel resonant tank circuit the coil can be thought of as an inductive load with a power factor correction ("PFC") capacitor connected across it. As connecting a capacitor would normally shift current and voltage 90 degrees out of phase to each other, the PFC capacitor is chosen with specifications to provide a reactive current flow equal and opposite to the large inductive current drawn by the coil. This cancels the phase shift effect and brings voltage and current back into phase for maximum power. Therefore, the only real current flow from the RF generator is the relatively small amount required to overcome losses in the PFC capacitor and coil.

Large eddy currents flowing on the surface of the induction tubes are required in order to heat the inductive microtubes. It is the job of the matching network and the coil itself to transfer a high voltage, low current from the RF generator to the low voltage, high current required to heat the induction tubes efficiently.

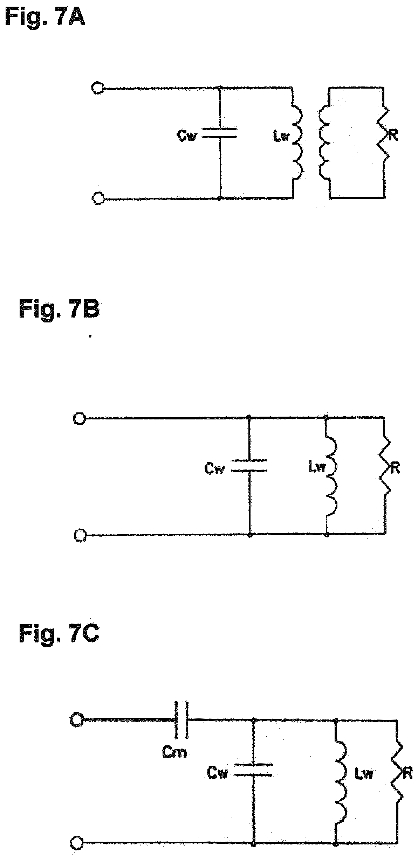

Referring to Fig. 7A, when thinking of the tank circuit incorporating the work coil (L_{W}) and its capacitor (C_{W}) as a parallel resonant circuit. The circuit has a resistance (R) due to the resistive vapor generator coupled into the work coil and due to the magnetic coupling between the two conductors. In practice, the resistance of the work coil, the resistance of the tank capacitor, and the reflected resistance of the vapor tubes all introduce a loss into the tank circuit and damp the resonance. Therefore it is useful to combine all the losses into a single "loss resistance."

In the case of a parallel resonant circuit, this loss resistance appears directly across the tank circuit. This resistance represents the only component that can consume real power, and therefore this can be considered as the load which power is being driven into in an efficient manner, as shown in Fig. 7B.

When driven at resonance, the current drawn by the tank capacitor and the work coil are equal in magnitude and opposite in phase and therefore cancel each other out as far as the source of power is concerned. This means that the only load seen by the RF generator at the resonant frequency is the loss resistance across the tank circuit. This loss resistance is quite low, however. The resistance of either the coil or capacitor alone would be low to begin with, and by being placed in parallel, the combined resistance is lowered even further. This places the combined load (coil and tank capacitor) even further away from the impedance for which the RF generator is optimized with its own impedance. For any RF generator attached to a real world load, the power delivered from the output terminal already has current and voltage out of phase, with the presumption that an operator will use a load closely matched to this RF generator impedance.

In the case of the vapor generator, the parallel resonant tank circuit is important, as it recirculates power that otherwise would exit the low impedance load and be reflected, but the low impedance of the combined load is even less matched to the generator, and recirculation of out-of-phase current and voltage takes them more out of phase.

The job of the C-matching network is to move delivered current and voltage back into phase and transform the relatively low loss resistance across the tank circuit up to a higher value that better suits the RF generator. This is as simple as placing the proper capacitor in series between the RF generator and coil.

Referring to Fig. 7C, the C-match network comprises capacitor Cₘ and Lₘ (not shown). Lₘ and L_{w} are in parallel, however, the work coil is assumed to be the combination of both inductors. C_{w} is adjusted for maximum voltage across the coil before Cₘ is connected to the circuit. Once connected, Cₘ is adjusted for the highest voltage across the coil.

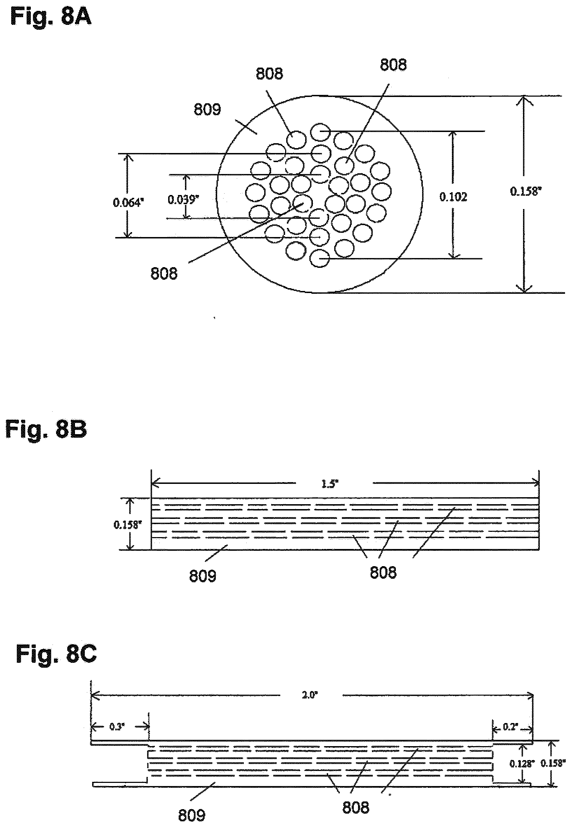

In the embodiments, described above, the generator is formed from outer and inner assemblies comprising hollow tubes. In an alternative embodiment, the generator can be constructed from a solid metal rod with holes drilled through the length of the rod, as shown in Figs. 8A-8C. Fig. 8A illustrates a cross sectional view of holes/tubes 808 drilled through the length of a solid metal rod 809, and Fig. 8B shows a side view of the rod. In the embodiment of Fig. 8C, the ends of the rod may be drilled out to form a proximal tubular space for hook up to a water line, and a distal tubular space for sintered filter, an expansion space, and a vapor line fitting. The solid metal rod with holes drilled through the length of the rod of Figs. 8A-8C can replace the microtubes of Fig. 1, for example.

Although particular embodiments of the present invention have been described above in detail, it will be understood that this description is merely for purposes of illustration and the above description of the invention is not exhaustive. Specific features of the invention are shown in some drawings and not in others, and this is for convenience only and any feature may be combined with another in accordance with the invention. A number of variations and alternatives will be apparent to one having ordinary skills in the art. The invention is defined by the following claims.

## Claims

1. An inductive vapor generator (100; 528), comprising:
an outer support assembly (104);
a coil (102) disposed around the outer support assembly (104);
an inner assembly (106) disposed within the outer support assembly (104);
a plurality of microtubes (108) disposed within the inner assembly (106), the microtubes (108) being configured to receive a fluid from a fluid source (118; 518); and
a RF generator electrically coupled to the coil (102), the RF generator being configured to apply a current to the coil (102) to inductively heat fluid in the plurality of microtubes (108) to convert the fluid into a heated condensable vapor;
**characterized in that** the inner assembly (106) includes a sintered filter (112).

2. The generator (100; 528) of claim 1, further comprising a first cap (110a) pressure-fit on a first end of the inner assembly (106) and a second cap (110b) pressure-fit on a second end of the inner assembly (106), wherein each of the first and second caps (110a, 110b) includes an o-ring seal (122).

3. The generator (100; 528) of claim 1, wherein the sintered filter (112) is configured to mechanically aerosolize the fluid as it enters the inner assembly (106) and impede backflow.

4. The generator (100; 528) of any one of claims 1 to 3, wherein the sintered filter (112) comprises a porous disc.

5. The generator (100; 528) of any one of claims 1 to 4, wherein the inner assembly (106) further includes a spacer tube (114), wherein the spacer tube (114) is positioned at least partially between the first cap (110b) and the microtubes (108).

6. The generator (100; 528) of any one of claims 1 to 5, wherein the o-ring seals (122) of the first and second caps (110a, 110b) are configured to mate with an internal surface of the internal assembly (106) to seal the internal assembly (106) off from the rest of the generator (100; 528).

7. The generator (100; 528) of any one of claims 1 to 6, wherein the second cap (110b) includes a lumen (116) to allow for the fluid to enter on a proximal end of the generator (100; 528) into the inner assembly (106) and wherein the first cap (110a) includes a lumen (116) to allow for the vapor to exit on a distal end of the generator (100; 528) from the internal assembly (106).

8. The generator (100; 528) of any one of the preceding claims wherein the outer support assembly (104) comprises a thermally insulating and electrically insulating tube.

9. The generator (100; 528) of claim 8 wherein the inner assembly (106) comprises a thermally conductive and electrically insulating tube.

10. The generator (100; 528) of claim 9 further comprising an air gap (116) between the outer support assembly (104) and the inner assembly (106).

11. The generator (100; 528) of any one of the preceding claims wherein the plurality of microtubes (108) comprises stainless steel microtubes or wherein the inner assembly (106) comprises a solid metal rod and the plurality of microtubes (108) comprises an array of channels drilled through the solid metal rod.

12. The generator (100; 528) of any one of the preceding claims wherein the outer support assembly (104) has a length of less than 70mm and/or a diameter of less than 10mm.

13. A medical vapor delivery device (500), comprising:
a handle (550);
a working shaft (570) coupled to the handle (550);
an induction vapor generator (100; 528) of any one of the preceding claims, wherein the generator (100; 528) is disposed in the handle (550) and fluidly coupled to a fluid source (118; 518) and the working shaft (570);
a controller (530) configured to apply a current to the coil (102) to convert a fluid in the plurality of microtubes (108) into a heated vapor inside the induction vapor generator (100; 528) for delivery of the heated vapor through the working shaft (570) to human tissue.

14. The device of claim 13 further comprising a treatment needle disposed near a distal portion of the working shaft.

## Patentansprüche

1. Induktiver Dampfgenerator (100; 528), der Folgendes umfasst:
eine äußere Stützanordnung (104);
eine Spule (102), die um die äußere Stützanordnung (104) herum angeordnet ist;
eine innere Anordnung (106), die innerhalb der äußeren Stützanordnung (104) angeordnet ist;
eine Vielzahl von Mikroröhren (108), die innerhalb der inneren Anordnung (106) angeordnet sind, wobei die Mikroröhren (108) dazu konfiguriert sind, ein Fluid aus einer Fluidquelle (118; 518) aufzunehmen; und
einen HF-Generator, der elektrisch mit der Spule (102) gekoppelt ist, wobei der HF-Generator dazu konfiguriert ist, einen Strom an die Spule (102) anzuwenden, um das Fluid in der Vielzahl von Mikroröhren (108) induktiv zu erwärmen und das Fluid in einen erwärmten, kondensierbaren Dampf umzuwandeln;
**dadurch gekennzeichnet, dass** die innere Anordnung (106) einen gesinterten Filter (112) beinhaltet.

2. Generator (100; 528) nach Anspruch 1, des Weiteren umfassend eine erste Kappe (110a), die auf ein erstes Ende der inneren Anordnung (106) aufgepresst ist, und eine zweite Kappe (110b), die auf ein zweites Ende der inneren Anordnung (106) aufgepresst ist, wobei jede von der ersten und zweiten Kappe (110a, 110b) eine O-Ring-Dichtung (122) beinhaltet.

3. Generator (100; 528) nach Anspruch 1, wobei der gesinterte Filter (112) dazu konfiguriert ist, das Fluid beim Eintritt in die innere Anordnung (106) mechanisch zu aerosolieren und einen Rückfluss zu verhindern.

4. Generator (100; 528) nach einem der Ansprüche 1 bis 3, wobei der gesinterte Filter (112) eine poröse Scheibe umfasst.

5. Generator (100; 528) nach einem der Ansprüche 1 bis 4, wobei die innere Anordnung (106) des Weiteren ein Distanzrohr (114) beinhaltet, wobei das Distanzrohr (114) mindestens teilweise zwischen der ersten Kappe (110b) und den Mikroröhren (108) angeordnet ist.

6. Generator (100; 528) nach einem der Ansprüche 1 bis 5, wobei die O-Ring-Dichtungen (122) der ersten und zweiten Kappen (110a, 110b) dazu konfiguriert sind, mit einer inneren Oberfläche der inneren Baugruppe (106) zusammenzupassen, um die innere Anordnung (106) gegenüber dem Rest des Generators abzudichten (100; 528) abzudichten.

7. Generator (100; 528) nach einem der Ansprüche 1 bis 6, wobei die zweite Kappe (110b) ein Lumen (116) beinhaltet, damit das Fluid an einem proximalen Ende des Generators (100; 528) in die innere Anordnung (106) eintreten kann, und wobei die erste Kappe (110a) ein Lumen (116) beinhaltet, damit der Dampf an einem distalen Ende des Generators (100; 528) aus der inneren Anordnung (106) austreten kann.

8. Generator (100; 528) nach einem der vorhergehenden Ansprüche, wobei die äußere Stützanordnung (104) ein thermisch isolierendes und elektrisch isolierendes Rohr umfasst.

9. Generator (100; 528) nach Anspruch 8, wobei die innere Anordnung (106) ein thermisch leitfähiges und elektrisch isolierendes Rohr umfasst.

10. Generator (100; 528) nach Anspruch 9, des Weiteren umfassend einen Luftspalt (116) zwischen der äußeren Stützanordnung (104) und der inneren Anordnung (106).

11. Generator (100; 528) nach einem der vorhergehenden Ansprüche, wobei die Vielzahl von Mikroröhren (108) Edelstahlmikroröhren umfasst oder wobei die innere Anordnung (106) einen massiven Metallstab umfasst und die Vielzahl von Mikroröhren (108) eine Reihe von Kanälen umfasst, die durch den massiven Metallstab gebohrt sind.

12. Generator (100; 528) nach einem der vorhergehenden Ansprüche, wobei die äußere Stützanordnung (104) eine Länge von weniger als 70 mm und/oder einen Durchmesser von weniger als 10 mm aufweist.

13. Medizinische Dampfabgabevorrichtung (500), die Folgendes umfasst:
einen Griff (550);
einen Arbeitsschaft (570), der mit dem Griff (550) gekoppelt ist;
einen induktiven Dampfgenerator (100; 528) nach einem der vorhergehenden Ansprüche, wobei der Generator (100; 528) in dem Griff (550) angeordnet und fluidisch mit einer Fluidquelle (118; 518) sowie dem Arbeitsschaft (570) gekoppelt ist;
einen Controller (530), der dazu konfiguriert ist, einen Strom an die Spule (102) anzuwenden, um ein Fluid in der Vielzahl von Mikroröhren (108) innerhalb des induktiven Dampfgenerators (100; 528) in erwärmten Dampf zur Abgabe des erwärmten Dampfes durch den Arbeitsschaft (570) an menschliches Gewebe umzuwandeln.

14. Vorrichtung nach Anspruch 13, des Weiteren umfassend eine Behandlungsnadel, die in der Nähe eines distalen Abschnitts des Arbeitsschafts angeordnet ist.

## Revendications

1. Générateur de vapeur par induction (100 ; 528), comprenant :
un ensemble de support externe (104) ;
une bobine (102) disposée autour de l'ensemble de support externe (104) ;
un ensemble interne (106) disposé à l'intérieur de l'ensemble de support externe (104) ;
une pluralité de microtubes (108) disposés à l'intérieur de l'ensemble interne (106), les microtubes (108) étant configurés pour recevoir un fluide en provenance d'une source de fluide (118 ; 518) ; et
un générateur RF couplé électriquement à la bobine (102), le générateur RF étant configuré pour appliquer un courant à la bobine (102) afin de chauffer par induction le fluide contenu dans la pluralité de microtubes (108) pour transformer le fluide en une vapeur condensable chauffée ;
**caractérisé en ce que** l'ensemble interne (106) comprend un filtre fritté (112).

2. Générateur (100 ; 528) selon la revendication 1, comprenant en outre un premier capuchon (110a) ajusté par pression sur une première extrémité de l'ensemble interne (106) et un second capuchon (110b) ajusté par pression sur une seconde extrémité de l'ensemble interne (106), dans lequel chacun des premier et second capuchons (110a, 110b) comprend un joint torique d'étanchéité (122).

3. Générateur (100 ; 528) selon la revendication 1, dans lequel le filtre fritté (112) est configuré pour transformer mécaniquement le fluide en aérosol lorsque celui-ci pénètre dans l'ensemble interne (106) et pour empêcher un reflux.

4. Générateur (100 ; 528) selon l'une quelconque des revendications 1 à 3, dans lequel le filtre fritté (112) comprend un disque poreux.

5. Générateur (100 ; 528) selon l'une quelconque des revendications 1 à 4, dans lequel l'ensemble interne (106) comprend en outre un tube d'espacement (114), dans lequel le tube d'espacement (114) est positionné au moins partiellement entre le premier capuchon (110b) et les microtubes (108).

6. Générateur (100 ; 528) selon l'une quelconque des revendications 1 à 5, dans lequel les joints toriques d'étanchéité (122) des premier et second capuchons (110a, 110b) sont configurés pour venir en contact par appariement avec une surface interne de l'ensemble interne (106) afin d'isoler de façon hermétique l'ensemble interne (106) vis-à-vis du reste du générateur (100 ; 528).

7. Générateur (100 ; 528) selon l'une quelconque des revendications 1 à 6, dans lequel le second capuchon (110b) comprend une lumière (116) pour permettre l'entrée du fluide, par une extrémité proximale du générateur (100 ; 528), à l'intérieur de l'ensemble interne (106) et dans lequel le premier capuchon (110a) comprend une lumière (116) pour permettre la sortie de la vapeur, par une extrémité distale du générateur (100 ; 528), hors de l'ensemble interne (106).

8. Générateur (100 ; 528) selon l'une quelconque des revendications précédentes, dans lequel l'ensemble de support externe (104) comprend un tube thermiquement isolant et électriquement isolant.

9. Générateur (100 ; 528) selon la revendication 8, dans lequel l'ensemble interne (106) comprend un tube thermiquement conducteur et électriquement isolant.

10. Générateur (100 ; 528) selon la revendication 9, comprenant en outre un entrefer (116) entre l'ensemble de support externe (104) et l'ensemble interne (106).

11. Générateur (100 ; 528) selon l'une quelconque des revendications précédentes, dans lequel la pluralité de microtubes (108) comprend des microtubes en acier inoxydable ou dans lequel l'ensemble interne (106) comprend une tige métallique pleine et la pluralité de microtubes (108) comprend un réseau de canaux percés à travers la tige métallique pleine.

12. Générateur (100 ; 528) selon l'une quelconque des revendications précédentes, dans lequel l'ensemble de support externe (104) présente une longueur inférieure à 70 mm et/ou un diamètre inférieur à 10 mm.

13. Dispositif médical de délivrance de vapeur (500), comprenant :
une poignée (550) ;
un arbre de travail (570) couplé à la poignée (550) ;
un générateur de vapeur par induction (100 ; 528) selon l'une quelconque des revendications précédentes, dans lequel le générateur (100 ; 528) est disposé dans la poignée (550) et est en communication fluidique avec une source de fluide (118 ; 518) et l'arbre de travail (570) ; et
un dispositif de commande (530) configuré pour appliquer un courant à la bobine (102) afin de transformer un fluide contenu dans la pluralité de microtubes (108) en une vapeur chauffée à l'intérieur du générateur de vapeur par induction (100 ; 528), dans le but de délivrer la vapeur chauffée sur un tissu humain par l'intermédiaire de l'arbre de travail (570).

14. Dispositif selon la revendication 13, comprenant en outre une aiguille de traitement disposée à proximité d'une partie distale de l'arbre de travail.
